# EUROPEAN PATENT APPLICATION

(11) **EP 2 371 368 A2**
(43) Date of publication of application: **05.10.2011**
(21) Application number: 11172000.9
(22) Date of filing: 28.05.2009
(51) Int. Cl.: A61K 31/575, A61P 17/06, A61P 17/12, A61P 17/00, A61P 35/00, A61K 31/56, A61K 47/10, A61K 47/26, A61K 47/44, A61K 9/00

(54) **Compositions and methods for treatment of inflammation and hyperkeratotic lesions**

(30) Priority: 30.05.2008 US 57696 P
(62) Divisional of application: 09767381.8
(71) Applicant: Novelix Pharmaceuticals, Inc., La Jolla CA 92037 (US)
(72) Inventor: The designation of the inventor has not yet been filed
(74) Representative: Goddard, Christopher Robert

(57) **Abstract**

A compound having the following formula, and related compounds, or a pharmaceutically acceptable salt thereof, for treating or preventing a disorder associated with aberrant proliferation of keratinocytes in the skin or mucous membranes, or associated with aberrant proliferation of basal cells in the skin or mucous membranes, excluding squamous cell carcinoma, in a mammal in need thereof:

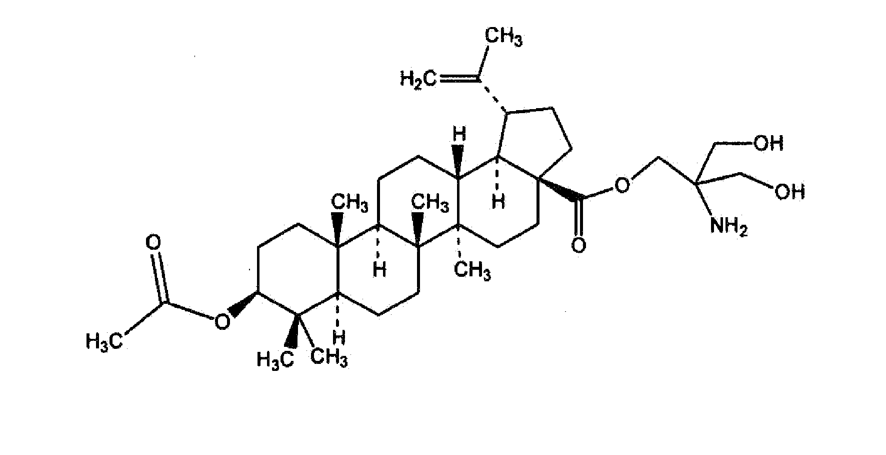

## Description

### FIELD OF THE INVENTION

The present invention relates to compositions and methods for treatment of inflammation and of hyperproliferative lesions of the skin and mucosal membranes. These lesions can have an inflammatory component and/or are hyperkeratotic, and may be benign, precancerous or cancerous, and. More specifically, the invention relates to the use of the betulinic acid derivative 3-O-acetylbetulinic acid - 2-amino-3-hydroxy-2-hydroxymethylpropyl ester (herein referred to as NVX-207) and related compounds, and to particular formulations comprising these compounds for the treatment and chemoprevention of inflammatory conditions, as well as conditions associated with aberrant proliferation of keratinoctyes or basal cells, such as psoriasis, actinic and non-actinic keratoses, squamous cell carcinoma and basal cell carcinoma.

### BACKGROUND OF THE INVENTION

Betulin is a naturally occurring triterpene isolated by extraction from birch bark and other plant sources. Betulin is easily converted to betulinic acid, and both compounds exhibit various biological activities, including anti-malaria, anti-inflammatory, anti-HIV and anti-tumor effects (Sami et al., Eur. J. Pharm. Sci. 29:1-13, 2006). Betulinic acid induces apoptosis in various tumor cell lines, including melanoma cells. Anti-melanoma effects have been observed both *in vitro* and *in vivo* in SCID mouse xenotranplantation models (Pisha et al., Nature Med. 1:1046-51, 1995). Betulinic acid was also shown to induce apoptosis in normal and malignant skin cells, including keratinocytes and melanocytes (Galgon T et al., Exp Dermatol. 14:736-43, 2005). U.S. Patent No. 7,312,205, the entire contents of which are incorporated herein by reference, describes various betulinic acid derivatives, and their use for treatment of HIV and various carcinomas, including melanomas, sarcomas, lymphomas, and squamous cell carcinomas.

Extracts of birch bark containing a mixture of betulin, betulinic acid and other terpenoids have been used to treat actinic keratosis (AK), which are cutaneous precancerous lesions of cytologically aberrant epidermal keratinocytes that develop in response to prolonged exposure to ultraviolet irradiation (Huyke et al., JDDG 4:132-137, 2006). In two clinical studies conducted by Huyke et al, patients were successfully treated with a standardized birch bark extract twice daily for two months (Huyke et al., JDDG 4:1320137, 2006 and Huyke et al., JDDG 7:128-133, 2009).

To date, there is no published evidence of betulinic acid or derivatives being used as mono-substances for the effective treatment of skin diseases in humans. A mono-substance formulation means a formulation having a single active agent (as used in single-agent therapy). The prevalence of AK is estimated to be 11-26% of the United States population, accounting for over 5 million physician visits per year. AK progresses to squamous cell carcinoma in about 1% to 16% of patients, emphasizing the need for prompt and effective treatment. Current standard treatment options for AK include surgical excision, laser treatment, cryotherapy, photodynamic therapy and topical application of various compounds including 5-fluorouracil, imiquimod and diclofenac/hylauronan gel. Many of these treatment methods have serious side effects, including scarring, pain, pigmentation changes or inflammatory reactions. For example, imiquimod (Aldara^{®}) and 5-fluorouracil (5-FU) cream (Efudex®, Carac®) can cause bleeding or scarring, resulting in poor patient acceptance, compliance and interruption of treatment. 5-FU topical treatment usually is associated with discomfort, pruritus, and burning at the sites of application, as well as erythema, erosion, crusting, and ulceration beginning at around the first week of the treatment. Treatment with imiquimod cream is also associated with adverse reactions, most notably local skin reactions such as erythema, scabbing, flaking, erosion, weeping, and vesicle formation. These reactions usually occur within the first and second treatment week. Because both 5-FU and imiquimod treatments have to be applied over several weeks in order to be effective, the above mentioned side-effects are usually of clinical relevance.

Psoriasis is an inflammatory, hyperkeratotic chronic skin disease, with a strong genetic basis, characterized by complex alterations in epidermal growth and differentiation and multiple biochemical, immunologic, and vascular abnormalities (Fitzpatrick's Dermatology in General Medicine, 7th Edition. Klaus Wolff, Lowell A. Goldsmith, Stephen I. Katz, Barbara A. Gilchrest, Amy S. Paller, David J. Leffell). Its root cause remains unknown. Psoriasis is widely considered to be a primary disorder of keratinocytes with a strong immunological background. Psoriasis affects approximately 2 percent of Americans and the prevalence ranges from 0.1 percent to 3 percent in various populations. Typical manifestations are erythematous scaly papules and plaques. Pustular and erythrodermic eruptions may also occur. Most common sites of involvement are scalp, elbows and knees, hands, feet, trunk, and nails.

The fully developed psoriatic lesion is characterized by capillary dilatation and a 10-fold increase in blood flow, numerous macrophages underlying the basement membrane, and increased numbers of dermal T cells (mainly CD4+) making contact with maturing dermal dendritic cells. The epidermis of the mature lesion manifests markedly increased (approximately 10-fold) keratinocyte hyperproliferation extending to the lower suprabasal layers, marked but not necessarily uniform loss of the granular layer with overlying compaction of the stratum corneum and parakeratosis, increased numbers of CD8+ T cells, and accumulation of neutrophils in the stratum corneum (Munro's microabscesses). The uninvolved near edge of the skin also shows an increased frequency of dyskeratotic keratinocytes. Keratinocytes are a major source of pro-inflammatory cytokines within the psoriatic lesion.

Other cell types, such as endothelial cells and fibroblasts, are also likely participants in the psoriatic pathogenic process. Endothelial cells are strongly activated in developing and mature lesions of psoriasis in addition to delivering a 10-fold increase in blood flow to the lesion, thereby playing a major role in facilitating the influx of leukocytes and serum proteins into psoriatic tissue. In addition, fibroblasts support keratinocyte proliferation in a paracrine manner, a process which is amplified in psoriasis. Furthermore, fibroblasts produce several chemotactic factors and support the migration of T cells out of psoriatic lesions. Thus, fibroblasts may also be intimately involved in psoriasis by directing the localization of T cells.

A broad spectrum of anti-psoriatic treatments, both topical and systemic, is available for the management of psoriasis. It is notable that most if not all of these treatments are immunomodulatory. When choosing a treatment regimen it is of importance to note that a recent study found that 40 percent of patients felt frustrated with the ineffectiveness of their current therapies, and 32 percent reported that treatment was not effective enough (Krueger G et al.: The impact of psoriasis on quality of life: Results of a 1998 National Psoriasis Foundation patient-membership survey (Archives Dermatology 137:280, 2001)).

Psoriasis is a chronic condition. It is therefore important to stress that the toxicity of a treatment during long-term use may be a limiting factor. Unfortunately, in most treatments, the duration of a treatment is restricted because of the cumulative toxicity potential of an individual treatment, and, in some instances, treatment efficacy may diminish with time (tachyphylaxis). Some treatments, such as calcipotriol, methotrexate (MTX), and acitretin, can be regarded as appropriate for continuous use. These treatments maintain efficacy and have low cumulative toxicity potential. In contrast, topical corticosteroids, dithranol, tar, photo(chemo)therapy, and cyclosporin are not indicated for continuous chronic use, and combinatorial or rotational treatments are suggested. However, patients with stable chronic plaque psoriasis who respond well to local treatments may not require a change of treatment. In cases of itchy/pruritic psoriasis, treatments with an irritative potential, such as dithranol, vitamin D3 analogues, and photo(chemo)therapy should be used cautiously, whereas treatments with potent anti-inflammatory effects, such as topical corticosteroids, are more appropriate.

AK and psoriasis are examples of hyperkeratotic disorders which have an inflammatory component. The inflammatory response is mediated by various cytokines (e.g., interleukin-1, interleukin-6, tumor necrosis factor (TNF)-α) and chemokines which are released by immune cells (e.g., neutrophils and macrophages). These pro-inflammatory cytokines result in inflammation, a process by which the body reacts to irritation or other injuries, and is characterized by one or more signs such as redness, warmth, swelling and pain.

Many other disorders known to have inflammatory components, include various forms of dermatitis such as rosacea and eczema, certain forms of alopecia, radiation and chemotherapy-induced mucositis, psoriasis and lupus.

One of the major obstacles for drug development of betulinic acid and its derivatives is their low solubility in polar solvents such as ethanol or in aqueous solutions. Most small animal models published to date utilize betulinic acid/betulinic acid derivative pharmaceutical formulations which are not well-suited for clinical use. Betulinic acid or its derivatives have not previously been used successfully as mono-substances in the treatment of AK or similar diseases. Extracts that are topically applied from the birch bark usually contain several potentially active compounds.

There is a clear need for improved and well tolerated treatment options for inflammatory disorders, as well as disorders associated with increased proliferative activity of the skin that are often associated with inflammation.

### SUMMARY OF THE INVENTION

The present invention provides a method of treating or preventing a disorder having an inflammatory component in a mammal in need thereof, by identifying a mammal in need of such treatment, and administering an effective amount of a compound having the following formula, a related compound, or pharmaceutically acceptable salt thereof, to the mammal:

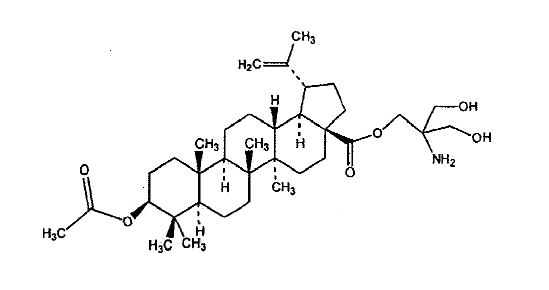

In one embodiment, the related compound has the following general formula: 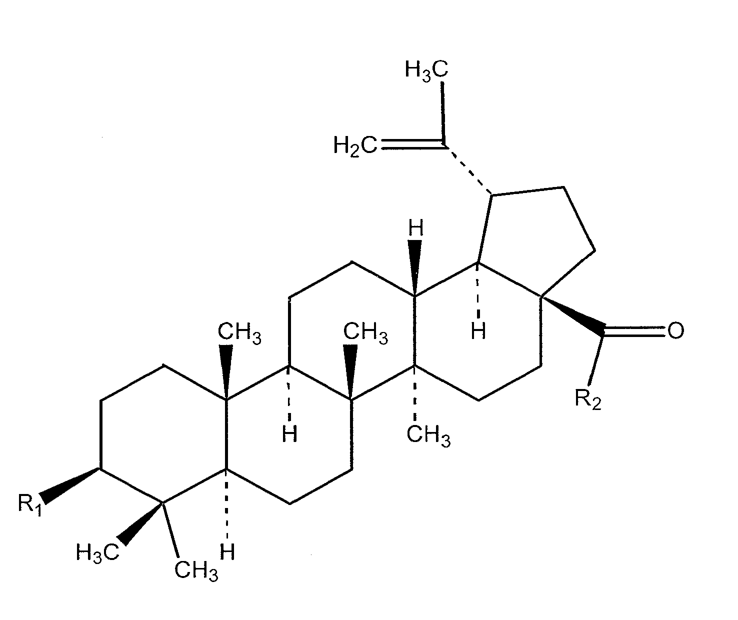 wherein R₁ represents a hydroxy group, an amino group, a protected hydroxy group or a protected amino group,
and R₂ is one of the following substituents:

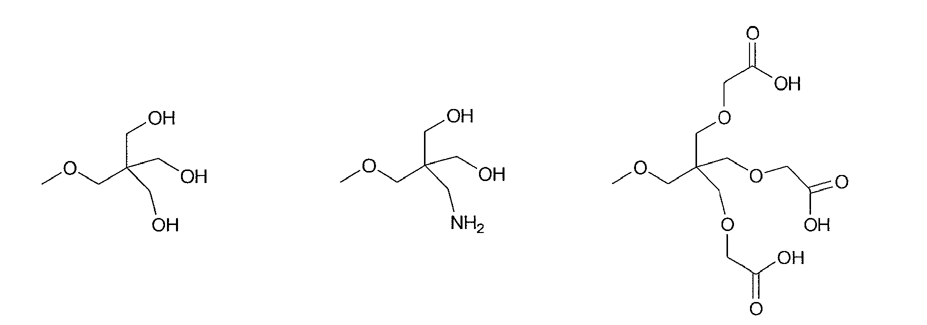

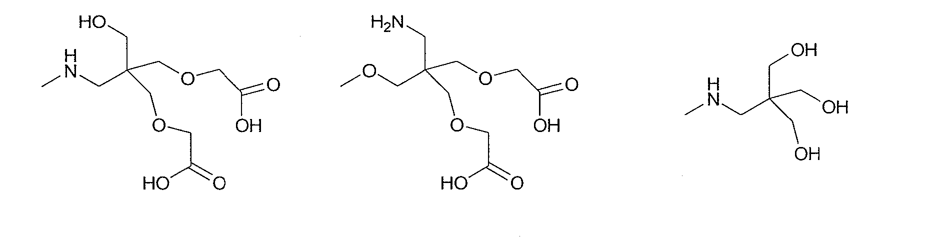

In another embodiment, R₁ represents a hydroxy group, an amino group or one of the following protected hydroxy or amino groups: 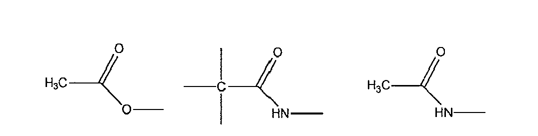 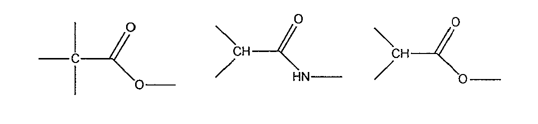 and R₂ is as defined above.

In one embodiment, the compound is administered topically. The topical administration may be with a cream, paste, gel, ointment, lotion, or in a transdermal delivery system (e.g., a patch applied to the skin). In another embodiment, the compound is administered to the oral cavity for the chemoprevention or treatment of precancerous oral lesions (e.g., oral leucoplakia, erythroplakia and oral sub mucous fibrosis). In the oral cavity, the compound contacts the oral mucosa, which is the mucous membrane epithelium of the mouth. The oral mucosa may be divided into three categories: masticatory mucosa (keratinized stratified squamous epithelium, found on the dorsum of the tongue, hard palate and attached gingival), lining mucosa (non-keratinized stratified epithelium, found almost everywhere else in the oral cavity), and specialized mucosa (taste bud regions on the dorsum of the tongue). The administration to the oral cavity may be with an oral rinse (e.g., mouthwash), oral gel, oral spray, oral paste, oral dressing, or toothpaste.

In other embodiments, the compound is administered intravenously, intramuscularly, subcutaneously, intralesionally, via aerosol, or orally.

In certain embodiments, the disorder is one or more of the following: : dermatitis, rosacea, eczema, alopecia, mucositis, psoriasis and lupus. The method involves treatment of mammals, particular humans, as well as other animals.

The present invention also provides a pharmaceutical formulation comprising the following compound, a related compound, or a pharmaceutically acceptable salt or polymorph thereof: 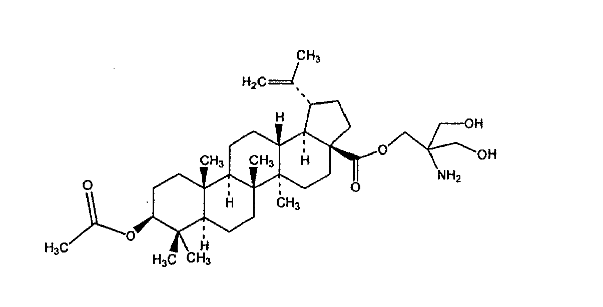 In formulations involving ethanol and/or DMSO or chemically similar solvents, the solvents can be present in an amount from 0.1 - 70 % (v/v); and a nonionic detergent in an amount from 0.1- 70 % (v/v). A non-ionic detergent can be present in an amount from 0.1-80 % (v/v).

In one embodiment, the nonionic detergent is Cremophor EL® (polyethoxylated castor oil), Tween® 80 (polyoxyethylen-sorbitan-monooleate), Tween® 40 (polyoxyethylene (20)-sorbitan-monopalmitate), Triton® X-100 (polyethylenglycol-[4-(1,1,3,3-tetramethylbutyl)phenyl]-ether), Span 20 (sorbitan monolaurate) and Span 85 (sorbitan trioleate). In another embodiment, the ethanol and/or DMSO are present in an amount of 20% (v/v). In another embodiment, the nonionic detergent is present in an amount of 20% (v/v).

The present invention also provides a method of treating or preventing a disorder associated with aberrant proliferation of skin or mucosal membrane keratinocytes or basal cells in a mammal in need thereof, comprising identifying a mammal in need of such treatment, and administering an effective amount of a compound having the following formula, a related compound, or a pharmaceutically acceptable salt thereof to the mammal:

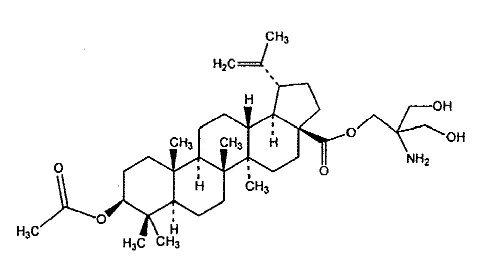

In one embodiment, the related compound has the following general formula: 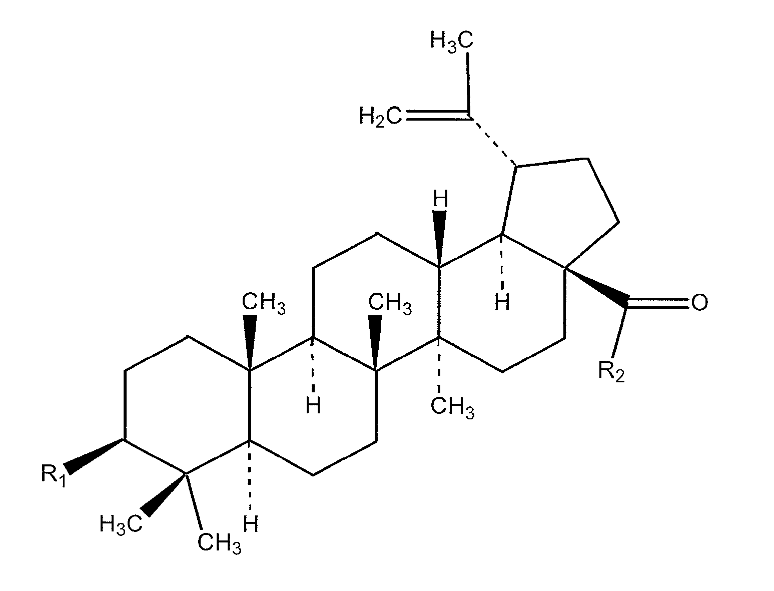 wherein R₁ represents a hydroxy group, an amino group, a protected hydroxy group or a protected amino group,
and R₂ is one of the following substituents:

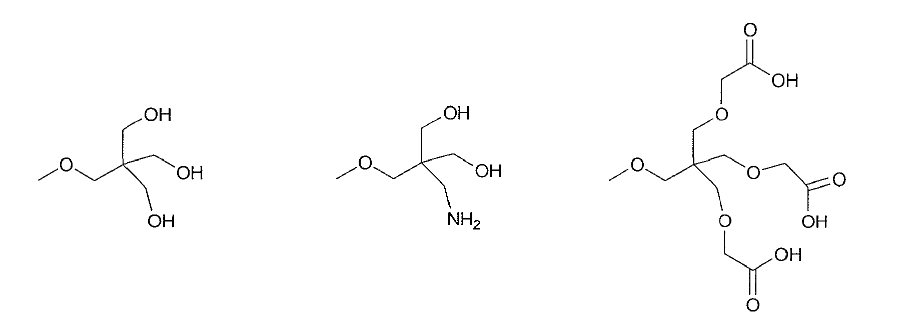

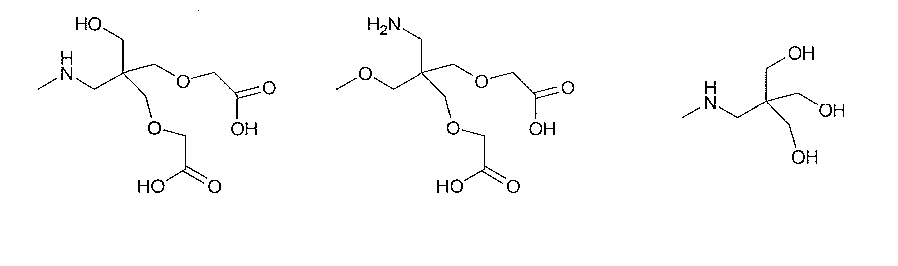

In another embodiment, R₁ represents a hydroxy group, an amino group or one of the following protected hydroxy or amino groups: 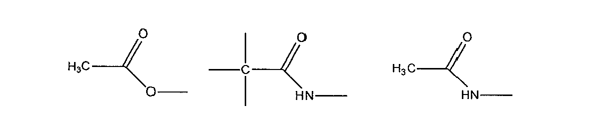 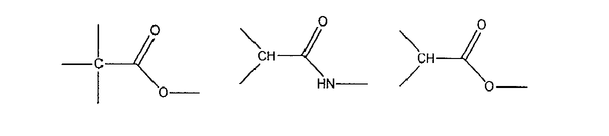 and R₂ is as defined above.

In one embodiment, the disorder is actinic keratosis, arsenical keratoses, thermal keratoses, hydrocarbon keratoses, chronic radiation keratoses, reactional keratoses, PUVA keratoses, viral keratoses, Bowenoid papulosis, epidermodysplasia verruciformis, erythroplasia of Queyrat, leukoplakia, erythroplakia, Bowen disease, squamous cell carcinoma, basal cell carcinoma or psoriasis. In another embodiment, the disorder is a hyperkeratotic lesion. In another embodiment, the mammal is a human. In another embodiment, the compound is administered topically. In other embodiments, the compound is formulated as a lotion, ointment, gel, cream, paste or in a transdermal delivery system. In another embodiment, the compound is administered to the oral cavity. In yet another embodiment, the compound is administered intravenously, intramuscularly, subcutaneously or via aerosol. In certain embodiments, the disorder is actinic keratosis or basal cell carcinoma or squamous cell carcinoma.

### BRIEF DESCRIPTION OF THE DRAWINGS

**Figures 1A-D** show cell survival in select human cancer cell lines: 518A2 melanoma (Fig. 1A), SW872 liposarcoma (Fig. 1B), Caco-2 colon carcinoma (Fig. 1C) and A549 lung carcinoma (Fig. ID). Cell survival as a percentage of untreated controls (y-axis) in response to NVX-207 (grey lines) or betulinic acid (black lines) in µg/ml (x-axis) are shown.

**Figures 2A-B** show a comparative time course analysis of survival of 518A2 melanoma cells in the presence of betulinic acid and NVX-207 (Fig. 2A). In Fig. 2B, the corresponding numbers are presented (mean ± S.E.).

**Figure 3** shows the effects of NVX-207 on about 80% confluent as well as on about 30 % confluent human endothelial (HUVEC), fibroblast and keratinocyte cell cultures. All cells were treated with 2.5 µM NVX-207. Percentage (means ± S.E., n=3) of control-treated cells are given. Mean survival in human umbilical vein endothelial cells (HUVEC) at 80% confluency (black bar) was 90.3 % (S.E. = 0.9) versus 18.3 % (S.E. = 3.9) at 30 % confluency (grey bar). Mean survival in NVX-207 treated fibroblasts at 80 % confluency was 92% (S.E. = 3.8) versus 3.8% mean survival (S.E. = 0.4) at 30 % confluency. In keratinocytes, mean survival (at 80 % confluency) was 92.7% (S.E. = 2.9) versus 17 % (S.E. = 4.3) in 30 % confluent cell cultures. HUVECs, keratinocytes and fibroblast cells were obtained from Lonza GmbH (Wuppertal, Germany) and cultivated according to the manufacturer's instructions.

### DETAILED DESCRIPTION OF THE PREFERRED EMBODIMENT

The present invention relates to the use of the betulinic acid derivative NVX-207, a related compound, or a pharmaceutically acceptable salt or polymorph thereof, and pharmaceutical compositions comprising one or more of these compounds, for preventing or treating a disorder having an inflammatory component (e.g., a disorder resulting from, or resulting in, inflammation) or a lesion arising from aberrant proliferation of skin or mucosal membrane keratinocytes (hyperkeratotic lesions) or basal cells. NVX-207 has the structure shown below: 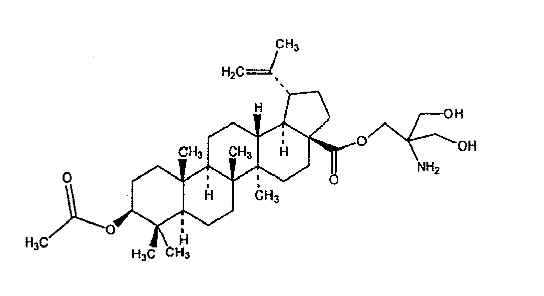 Disorders having an inflammatory component and/or those associated with hyperproliferation of certain epidermal cells such as keratinocytes or basal cells include benign, pre-malignant and malignant diseases such as actinic keratosis, squamous cell carcinoma and basal cell carcinoma of the skin. It should be noted that there may be overlap between inflammation and aberrant proliferation of epidermal cells, in that a disorder may be characterized by both of these processes. For example, actinic keratosis is associated with both inflammation and aberrant keratinocyte proliferation. The same is the case for psoriasis, squamous cell carcinoma, certain forms of basal cell carcinoma and rosacea. The treatment of any disorder that is characterized by, or results in, either or both of these features, with the compounds and methods disclosed herein is within the scope of the present invention.
In other embodiments, the hyperakeratotic disease may be one or more of the following lesions associated with altered keratinocyte or basal cell biology: actinic keratoses, arsenical keratoses, thermal keratoses, hydrocarbon keratoses, chronic radiation keratoses, reactional keratoses, PUVA keratoses, viral keratoses, Bowenoid papulosis, epidermodysplasia verruciformis, erythroplasia of Queyrat, leukoplakia and erythroplakia, Bowen disease or squamous cell carcinoma in situ, squamous and basal cell carcinoma of the skin, and psoriasis. Another embodiment relates to NVX-207, a related compound, or a pharmaceutically acceptable salt or polymorph thereof, for use in treatment of any of the disorders disclosed herein. Another embodiment relates to the use of NVX-207, a related compound, or a pharmaceutically acceptable salt or polymorph thereof, in the preparation of a medicament for treatment of any of the disorders disclosed herein.
In addition many other disorders are known to have inflammatory components, and can also be treated using the compounds and methods described herein. These include various forms of dermatitis such as rosacea and eczema, certain forms of alopecia, radiation and chemotherapy-induced mucositis, psoriasis and lupus.

Related compounds are disclosed in US Patent No. 7,312,205, the entire contents of which are incorporated herein by reference. The synthesis and structure of NVX-207 and related compounds are described in U.S. Patent No. 7,312,205, The compound may be formulated as a pharmaceutical composition, and may include one or more physiologically acceptable carriers or diluents.

As used herein the term "a related compound" encompasses any of the betulinic acid derivatives disclosed in US Patent No. 7,312,205 which have the following general formula: 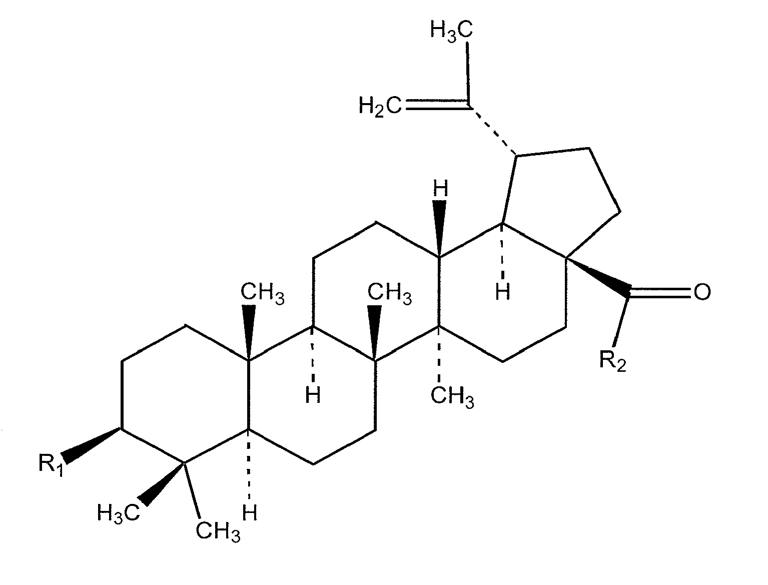 wherein R₁ represents a hydroxy group, an amino group, a protected hydroxy group or a protected amino group. Suitable protective groups are described in, for example, Chapters 2 and 7 of "Protective Groups in Organic Synthesis", T.W. Greene and P.G.M. Wuts, 3rd Edition, John Wiley & Sons, Inc. (1999), the disclosure of which is incorporated herein by reference,
and R₂ is one of the following substituents:

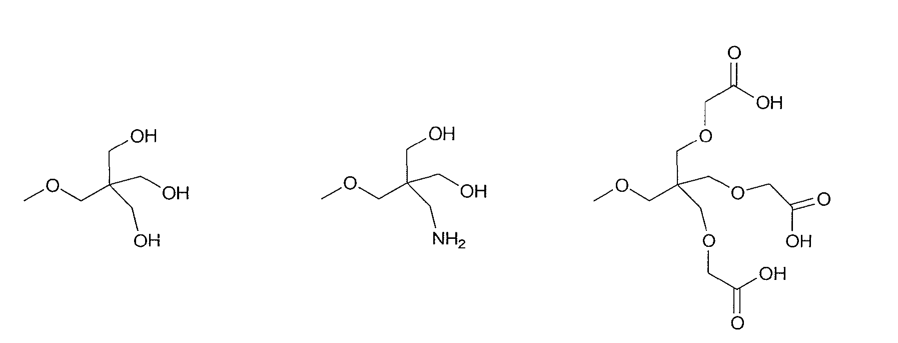

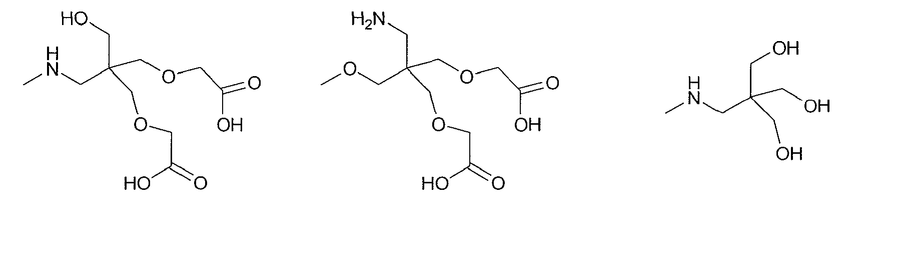

Other compounds related to NVX-207 having the general formula (I) as indicated above, suitable for use in the compositions and methods described herein are those in which R₁ represents a hydroxy group, an amino group or one of the following protected hydroxy or amino groups: 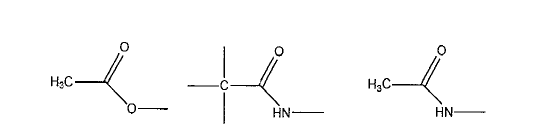 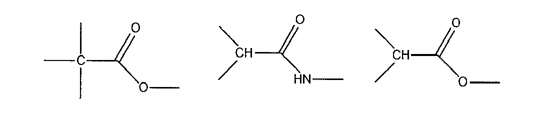 and R₂ is as defined above.

NVX-207 or a related compound can be used alone, or may be combined with one or more compounds such as imiquimod and 5-fluorouracil conventionally used to treat such conditions. In addition, NVX-207 or a related compound can be combined with one or more conventional anti-inflammatory agents to treat a disorder having an inflammatory component. Suitable anti-inflammatory agents include steroidal (e.g., dexamethasone, prednisone) and non-steroidal agents (NSAIDs). Examples of NSAIDs include ibuprofen, acetaminophen, naproxen, indomethacin, ketoprofen and diclofenac.

The use of NVX-207 or a related compound allows reduction of the required amount of the other compound(s), thus reducing the likelihood of side effects of the other compound(s).

Some hyperkeratotic lesions, such as actinic keratosis and psoriasis, have inflammatory components and are characterized by inflammation. Inflammation is also a hallmark of other skin conditions such as rosacea. As described herein, NVX-207, a related compound, or a pharmaceutically acceptable salt thereof, can be used to treat these inflammatory disorders, and do not result in inflammation at the treatment site. Thus, in one embodiment, NVX-207, a related compound, or a pharmaceutically acceptable salt thereof, is used to reduce inflammation in an individual in need thereof. In one embodiment, NVX-207, a related compound, or a pharmaceutically acceptable salt thereof, is used to treat an inflammatory skin disorder by administering a composition containing this compound to an individual.

Patients who can benefit from chemoprevention measures by NVX-207 or a related compound include those who are at high risk of developing skin cancer. These patients should undergo continuous prevention measures including frequent dermatologic examinations. These are important to detect new and recurrent skin cancers and to accurately document the numbers of new cancers and their locations. If new lesions are developing at a rapid rate, examinations may be required as frequently as every 2 weeks. Lesions that are suspicious for skin cancer should be biopsi*ed. Unfortunately, in some patients, the presence of large numbers of hyperkeratotic lesions like actinic keratoses can create a background where there are so many lesions that it is difficult to choose which lesions are most suspicious and, therefore, most in need of biopsy and of subsequent treatment. In these patients, NVX-207 treatment reduces or improves hyperkeratosis and thereby prevents the risk for development of skin cancer.

Examples of criteria that may be used to select patients for NVX-207 or a related compound skin cancer chemoprevention include: (1) individuals who are actively developing large numbers of new tumors; (2) individuals undergoing aggressive surveillance for new skin cancers and standard management of cancers identified; (3) individuals that need to utilize extensive sun protection strategies based on their skin type, and (4) individuals having concurrent keratotic lesions of, for example, viral origin (e.g., HPV), AK, etc., making diagnosis of newly developing carcinoma of the skin difficult.

Compared to betulinic acid, NVX-207 unexpectedly exhibits an over 20-fold increase in solubility in DMSO and an over 300-fold increase in solubility in ethanol and, as described below, exhibits an up to 5-fold greater efficacy on a molar basis in promoting cell death of various types of cancer cells and non-cancer cells in human and animal cell lines of varying histology.

After solution in ethanol or DMSO, or chemically similar solvents, NVX-207 or a related compound may be formulated with co-solvents such as Cremophor EL® and/or Tween® 80, in a similar manner described for taxanes. For example, the formulation approach for paclitaxel (TAXOL®; Bristol-Myers Squibb) was to use 50% Cremophor EL® and 50% ethanol. The pharmaceutical formulation of paclitaxel contains 30 mg paclitaxel dissolved in 5 ml of this (1:1, v/v) mixture. These and equivalent formulations allow for the application of NVX-207 in sufficiently high concentrations not only for topical, but also for intralesional and systemic (intravenous infusion) use. Solubility of NVX-207 in ethanol was determined to be at least 300 mg/ml and reached 250 mg/ml in DMSO. Solubility of betulinic acid in ethanol (< 1 mg/ml) as well as in DMSO (10 mg/ml) is, by comparison, significantly lower. The solubility of NVX-207 in ethanol-Tween® 80 was evaluated. Two-hundred mg of NVX-207 was dissolved in 1 ml ethanol. The solution appeared to be clear and showed a light yellow color. Then an equal volume of Tween® 80 was added, to achieve a stock solution of 100 mg/ml of NVX-207. The stock solution was clear and yellow. Each stock solution was diluted first 1:1 (50 mg/ml NVX-207), then 1:5 (20 mg/ml NVX-207) and finally 1:10 (10 mg/ml NVX-207) with water. The final diluted solutions remained clear. NVX-207 therefore has, apart from its higher activity, significant advantages over betulinic acid, that include higher activity, better solubility in pharmaceutically suitable solvents, and better handling.

NVX-207 (3-O-acetylbetulinic acid - 2-amino-3-hydroxy-2-hydroxymethylpropyl ester) is a compound having the following structure:

The term "pharmaceutical composition" refers to a mixture of NVX-207, or a pharmaceutically acceptable salt thereof, with other chemical components, such as diluents or carriers. The pharmaceutical composition facilitates administration of the compound to an organism. Multiple techniques of administering a compound exist in the art including, but not limited to, oral, injection, aerosol, parenteral, and topical administration. Pharmaceutical compositions can also be obtained by reacting compounds with inorganic or organic acids such as hydrochloric acid, hydrobromic acid, sulfuric acid, nitric acid, phosphoric acid, methanesulfonic acid, ethanesulfonic acid, p-toluenesulfonic acid, salicylic acid and the like.

The term "carrier" defines a chemical compound that facilitates the incorporation of a compound into cells or tissues. For example dimethyl sulfoxide (DMSO) is a commonly utilized carrier as it facilitates the uptake of many organic compounds into the cells or tissues of an organism.

The term "diluent" defines chemical compounds diluted in water that will dissolve the compound of interest as well as stabilize the biologically active form of the compound. Salts dissolved in buffered solutions are utilized as diluents in the art. One commonly used buffered solution is phosphate buffered saline because it mimics the salt conditions of human blood. Since buffer salts can control the pH of a solution at low concentrations, a buffered diluent rarely modifies the biological activity of a compound.

The term "physiologically acceptable" defines a carrier or diluent that does not abrogate the biological activity and properties of the compound.

The term "pharmaceutically acceptable salt" refers to a formulation of a compound that does not cause significant irritation to an organism to which it is administered and does not abrogate the biological activity and properties of the compound. Pharmaceutical salts can be obtained by reacting a compound of the invention with inorganic acids such as hydrochloric acid, hydrobromic acid, sulfuric acid, nitric acid, phosphoric acid, methanesulfonic acid, ethanesulfonic acid, p-toluenesulfonic acid, salicylic acid and the like. Pharmaceutical salts can also be obtained by reacting a compound of the invention with a base to form a salt such as an ammonium salt, an alkali metal salt, such as a sodium or a potassium salt, an alkaline earth metal salt, such as a calcium or a magnesium salt, a salt of organic bases such as dicyclohexylamine, N-methyl-D-glutamine, tris(hydroxymethyl)methylamine, and salts with amino acids such as arginine, lysine, and the like.

The term "metabolite" refers to a compound to which NVX-207 is converted within the cells of a mammal. The pharmaceutical compositions of the present invention may include a metabolite of NVX-207 instead of NVX-207. The scope of the methods of the present invention includes those instances where NVX-207 is administered to the patient, yet the metabolite is the bioactive entity.

In a further aspect, the present invention relates to a method of treating a patient with a pharmaceutical composition as described herein.

The term "treating" or "treatment" does not necessarily mean total cure. Any alleviation of any undesired signs or symptoms of the disease to any extent or the slowing down of the progress of the disease can be considered treatment. Furthermore, treatment may include acts that may worsen the patient's overall feeling of well being or appearance.

The pharmaceutical compositions comprising NVX-207 or a related compound described herein can be administered to an animal, such as a mammal (e.g., dog, cat, horse, pig, cow, goat, sheep) or human *per se*, or in pharmaceutical compositions where they are mixed with other active ingredients, as in combination therapy, or suitable carriers or excipient(s). Techniques for formulation and administration of the compounds of the instant application can be found in "Remington's Pharmaceutical Sciences," Mack Publishing Co., Easton, PA, 18th edition, 1990.

Suitable routes of administration include, for example, oral, rectal, topical, transmucosal, oral mucosal or intestinal administration; parenteral delivery, including intralesional, intramuscular, subcutaneous, intravenous, intramedullary injections, as well as intrathecal, direct intraventricular, intraperitoneal, intranasal, or intraocular injections.

Furthermore, one can administer the drug in a targeted drug delivery system, for example, in a liposome coated with a tissue-specific antibody. The liposomes will be targeted to and taken up selectively by the tissue.

The pharmaceutical compositions of the present invention can be manufactured in a manner that is itself known, e.g., by means of conventional mixing, dissolving, granulating, dragee-making, levigating, emulsifying, encapsulating, entrapping or tabletting processes.

Pharmaceutical compositions for use in accordance with the present invention thus can be formulated in conventional manner using one or more physiologically acceptable carriers comprising excipients and auxiliaries which facilitate processing of the active compounds into preparations which can be used pharmaceutically. Proper formulation is dependent upon the route of administration chosen. Any of the well-known techniques, carriers, and excipients can be used as suitable and as understood in the art; e.g., in Remington's Pharmaceutical Sciences, above.

For injection, NVX-207 or a related compound can be dissolved in aqueous solutions after formulation with Tween® 80, Cremophor EL®, or similar detergents, preferably in physiologically compatible buffers such as Hanks's solution, Ringer's solution, or physiological saline buffer. Similar formulation techniques as those used for the taxanes can be utilized here. For transmucosal administration, penetrants appropriate to the barrier to be permeated are used in the formulation. Such penetrants are generally known in the art.

In addition, NVX-207 or a related compound can be dissolved in aqueous solutions after formulation in cyclodextrins.

For oral administration, NVX-207 or a related compound can be formulated readily by combining the active compound with pharmaceutically acceptable carriers well known in the art. Such carriers enable the compounds of the invention to be formulated as tablets, pills, dragees, capsules, liquids, gels, syrups, slurries, suspensions and the like, for oral ingestion by a patient to be treated. Pharmaceutical preparations for oral use can be obtained by mixing one or more solid excipient with the pharmaceutical combination of the invention, optionally grinding the resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate.

For topical administration, NVX-207 or a related compound can be formulated for administration to the epidermis as solutions, emulsions, ointments, gels, creams, pastes, salves, sunscreens, or lotions, shampoos, or formulated in a topical or transdermal delivery system.

A transdermal or topical delivery system involves a bandage or dressing designed to permit passage of a medicament to the skin or through the skin (by absorption therethrough) without preliminary puncture or abrasion of a living body. Transdermal drug delivery involves the delivery of drugs through the skin. Topical drug delivery systems apply formulations to the skin with the goal of localized application and minimal systemic circulation. With topical drug delivery systems, the active drug does not enter the bloodstream. Guidance for combining NVX-207 with transdermal or topical delivery systems for application of a drug to the skin to achieve therapeutic effects are disclosed in detail in generally available references (Topical Drug Bioavailablity, Bioequivalence, and Penetration (eds. Vinod P. Shah and Howard I. Maibach, Plenum Press, 1993), and in Skin Barrier, Principles of Percutaneous Absorption (Hans Schaefer and Thomas E. Redelmeier, Karger Publ., 1996; Benson, Heather A.E., Transdermal Drug Delivery: Penetration Enhancement Techniques, Current Drug Delivery 2:23-33 (2005); Topical Drug Delivery Formulations edited by David W. Osborne and Anton H. Amann (available at http://chipsbooks.com/topdrug.htm); Hadgraft, Jonathanl, Lane, Majella E., Passive Transdermal Drug Delivery Systems: Recent Considerations and Advances, American Journal of Drug Delivery, Volume 4, Number 3, 2006 , pp. 153-160(8)).

Ointments and creams can, for example, be formulated with an aqueous or oily base with the addition of suitable thickening and/or gelling agents. Lotions can be formulated with an aqueous or oily base and will in general also contain one or more emulsifying agents, stabilizing agents, dispersing agents, suspending agents, thickening agents, or coloring agents.

In another embodiment, the compound is administered to the oral cavity for the chemoprevention or treatment of precancerous oral lesions (e.g., oral leucoplakia, erythroplakia and oral submucous fibrosis). In the oral cavity, the compound is delivered so as to contact the oral mucosa at the site(s) of precancerous lesions. The administration to the oral cavity may be with an oral rinse (e.g. mouth wash), oral gel, oral spray, oral paste, or toothpaste.

One preferred topical formulation comprises NVX-207 or a related compound and ethanol and/or DMSO, and one or more non-ionic detergents, including Cremophor EL® (polyethoxylated castor oil), Tween® 80 (polyoxyethylen-sorbitan-monooleate), Tween® 40 (polyoxyethylene (20)-sorbitan-monopalmitate), Triton® X-100 (polyethylenglycol-[4-(1,1,3,3-tetramethylbutyl)phenyl]-ether), Span® 20 (sorbitan monolaurate) and Span® 85 (sorbitan trioleate).

The combined amount of ethanol and/or DMSO can range from 0.1 % to 70 % (v/v). In one embodiment, about 20% (v/v) ethanol and/or DMSO is/are used. The total amount of nonionic detergent(s) can range from 0.1 % to 80 % (v/v). In one embodiment, about 20% (v/v) nonionic detergent is used.

The compound may be delivered to the oral cavity via dragee cores, which are provided with suitable coatings. For this purpose, concentrated sugar solutions can be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments can be added to the tablets or dragee coatings for identification or to characterize different combinations of active compound doses.

Pharmaceutical preparations which can be used orally, including sublingually, which include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds can be dissolved or suspended in suitable liquids, such as fatty oils, liquid paraffin, or liquid polyethylene glycols. In addition, stabilizers can be added. All formulations for oral administration should be in dosages suitable for such administration.

For buccal administration, the compositions can take the form of tablets or lozenges formulated in conventional manner.

For administration by inhalation, the compounds for use according to the present invention are conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide, or other suitable gas. In the case of a pressurized aerosol the dosage unit can be determined by providing a valve to deliver a metered amount. Capsules and cartridges of, e.g., gelatin for use in an inhaler or insufflator can be formulated containing a powder mix of the compound and a suitable powder base such as lactose or starch.

The compounds can be formulated for parenteral administration by injection, e.g., by bolus injection or continuous infusion. Formulations for injection can be presented in unit dosage form, e.g., in ampoules or in multi-dose containers, with an added preservative. The compositions can take such forms as suspensions, solutions or emulsions in oily or aqueous vehicles, and can contain formulatory agents such as suspending, stabilizing and/or dispersing agents.

Pharmaceutical formulations for parenteral administration include aqueous solutions of the active compounds in water-soluble form. Additionally, suspensions of the active compounds can be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions can contain substances which increase the viscosity of the suspension, such as sodium carboxymethyl cellulose, sorbitol, or dextran. Optionally, the suspension can also contain suitable stabilizers or agents which increase the solubility of the compounds to allow for the preparation of highly concentrated solutions.

Alternatively, the active ingredient can be in powder form for constitution with a suitable vehicle, *e.g.*, sterile pyrogen-free water, before use.

The compounds can also be formulated in rectal compositions such as suppositories or retention enemas, *e.g.*, containing conventional suppository bases such as cocoa butter or other glycerides.

In addition to the formulations described previously, the compounds can also be formulated as a depot preparation. Such long acting formulations can be administered by implantation (for example subcutaneously or intramuscularly) or by intramuscular injection. Thus, for example, the compounds can be formulated with suitable polymeric or hydrophobic materials (for example, as an emulsion in an acceptable oil) or ion exchange resins, or as sparingly soluble derivatives, for example, as a sparingly soluble salt.

One example of a pharmaceutical carrier for the hydrophobic compounds of the invention is a co-solvent system comprising benzyl alcohol, a non-polar surfactant, a water-miscible organic polymer, and an aqueous phase. A common co-solvent system used is the VPD co-solvent system, which is a solution of 3% w/v benzyl alcohol, 8% w/v of the non-polar surfactant Tween® 80, and 65% w/v polyethylene glycol 300, made up to volume in absolute ethanol. Naturally, the proportions of a co-solvent system can be varied considerably without destroying its solubility and toxicity characteristics. Furthermore, the identity of the co-solvent components can be varied: for example, other low-toxicity non-polar surfactants may be used instead Tween® 80; the fraction size of polyethylene glycol can be varied; other biocompatible polymers can replace polyethylene glycol, e.g., polyvinyl pyrrolidone; and other sugars or polysaccharides can substitute for dextrose.

Alternatively, other delivery systems for hydrophobic pharmaceutical compounds can be employed. Liposomes and emulsions are well known examples of delivery vehicles or carriers for hydrophobic drugs. Certain organic solvents such as dimethylsulfoxide also can be employed, although usually at the cost of greater toxicity. Additionally, the compounds may be delivered using a sustained-release system, such as semi-permeable matrices of solid hydrophobic polymers containing the therapeutic agent. Various sustained-release materials have been established and are well known by those skilled in the art. Sustained-release capsules may, depending on their chemical nature, release the compounds for a few weeks up to over 100 days.

Many of the compounds used in the pharmaceutical combinations of the invention can be provided as salts with pharmaceutically compatible counter-ions. Pharmaceutically compatible salts can be formed with many acids, including but not limited to hydrochloric, sulfuric, acetic, lactic, tartaric, malic, succinic, *etc*. Salts tend to be more soluble in aqueous or other protonic solvents than are the corresponding free acid or base forms.

Pharmaceutical compositions suitable for use in the present invention include compositions where the active ingredients are contained in an amount effective to achieve its intended purpose. More specifically, a therapeutically effective amount means an amount of compound effective to prevent, alleviate or ameliorate symptoms of disease or prolong the survival of the subject being treated. Determination of a therapeutically effective amount is well within the capability of those skilled in the art, especially in light of the detailed disclosure provided herein.

The exact formulation, route of administration and dosage for the pharmaceutical compositions of the present invention can be chosen by the individual physician in view of the patient's condition (See *e.g.*, Fingl *et al.* 1975, in "The Pharmacological Basis of Therapeutics", Ch. 1 p. 1). Typically, the dose range of the composition administered to the patient can be from about 0.5 to 1000 mg/kg of the patient's body weight. The dosage may be a single one or a series of two or more given in the course of one or more days, as is needed by the patient. Note that for almost all of the specific compounds mentioned in the present disclosure, human dosages for treatment of at least some condition have been established. Thus, in most instances, the present invention will use those same dosages, or dosages that are between about 0.1% and 500%, more preferably between about 25% and 250% of the established human dosage. Where no human dosage is established, as will be the case for newly-discovered pharmaceutical compounds, a suitable human dosage can be inferred from ED₅₀ or ID₅₀ values, or other appropriate values derived from *in vitro* or *in vivo* studies, as qualified by toxicity studies and efficacy studies in animals.

Although the exact dosage will be determined on a drug-by-drug basis, in most cases, some generalizations regarding the dosage can be made. The daily dosage regimen for an adult human patient can be, for example, a topical dose of between 0.01 mg and 10 mg of each ingredient, preferably between 1 mg and 10 mg. The daily dosage regimen for an adult human patient can be, for example, an oral dose of between 10 mg and 1000 mg of each ingredient, preferably between 10 mg and 500 mg, e.g. 25 to 500 mg or an intralesional or subcutaneous dose of each ingredient between 0.01 mg and 100 mg, preferably between 0.1 mg and 60 mg, e.g. 1 to 40 mg of each ingredient of the pharmaceutical compositions of the present invention or a pharmaceutically acceptable salt thereof calculated as the free base, the composition being administered, for example, 1 to 4 times per day. Alternatively the compositions of the invention can be administered by continuous intravenous infusion, preferably at a dose of each ingredient up to 400 mg per day. Thus, the total daily dosage by oral administration of each ingredient will typically be in the range 1 to 2500 mg and the total daily dosage by parenteral administration will typically be in the range 0.1 to 400 mg. Suitably the compounds will be administered for a period of continuous therapy, for example for a week or more, or for months or years. In one embodiment, the formulations (as described above) for administration to the oral mucosa are administered every day, every other day, or every third day, one to three times per day for one week, two weeks, three weeks, one month, several months, one year or longer.

Dosage amount and interval may be adjusted individually to provide plasma levels of the active moiety which are sufficient to maintain the modulating effects, or minimal effective concentration (MEC). The MEC will vary for each compound but can be estimated from in vitro data. Dosages necessary to achieve the MEC will depend on individual characteristics and route of administration. However, HPLC assays can be used to determine plasma concentrations.

Dosage intervals can also be determined using MEC value. Compositions should be administered using a regimen that maintains plasma levels above the MEC for 10-90% of the time, preferably between 30-90% and most preferably between 50-90%.

In cases of local administration or selective uptake, the effective local concentration of the drug may not be related to plasma concentration.

The amount of composition administered will, of course, be dependent on the subject being treated, on the subject's weight, the severity of the affliction, the manner of administration and the judgment of the prescribing physician.

The compositions can, if desired, be presented in a pack or dispenser device which may contain one or more unit dosage forms containing the active ingredient. The pack can, for example, comprise metal or plastic foil, such as a blister pack. The pack or dispenser device may be accompanied by instructions for administration. The pack or dispenser can also be accompanied with a notice associated with the container in form prescribed by a governmental agency regulating the manufacture, use, or sale of pharmaceuticals, which notice is reflective of approval by the agency of the form of the drug for human or veterinary administration. Such notice, for example, can be the labeling approved by the U.S. Food and Drug Administration for prescription drugs, or the approved product insert. Compositions comprising a compound of the invention formulated in a compatible pharmaceutical carrier can also be prepared, placed in an appropriate container, and labeled for treatment of an indicated condition.

The compositions described herein can also be used in the preparation of a medicament for treatment of any of the disorders described above.

### Example 1

### Preparation of NVX-207

NVX-207 was prepared as described in U.S. Patent No. 7,312,205. The reaction scheme is shown below, in which IV is NVX-207.

### 1) Acetyl betulic acid-2-amino-3-hydroxy-2-hydroxymethyl propyl ester IV (Compound B)

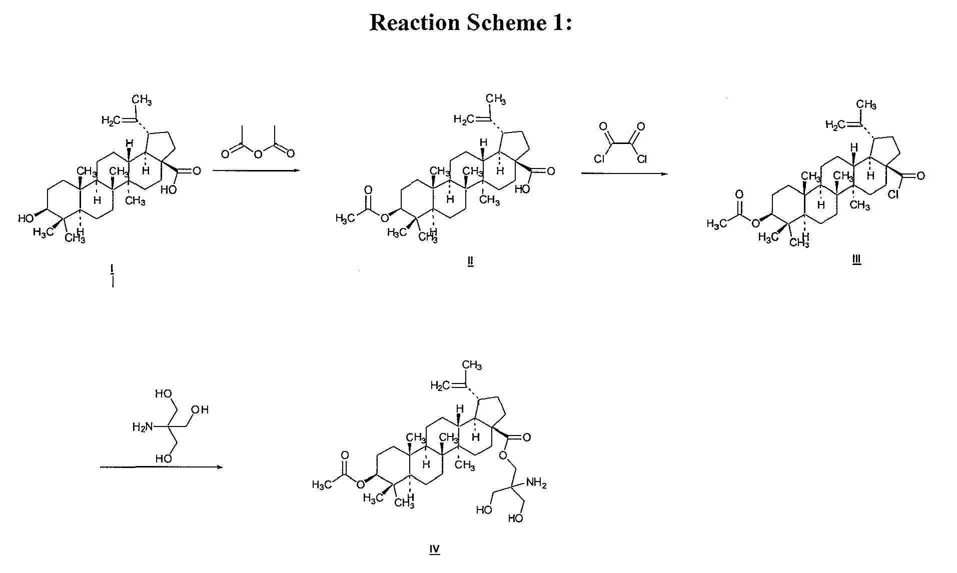

The synthesis of acetyl betulinic acid-2-amino-3-hydroxy-2-hydroxymethyl propyl ester IV is performed by departing from betulinic acid I via the intermediate stages of acetyl betulinic acid II and the respective acid chloride III, by reacting the acid chloride III with trishydroxymethylaminomethane.

### a) Acetyl betulinic acid (MW 498.74) II

Two grams of betulinic acid I (MW 456.70) in 50 ml acetic anhydride are heated at reflux for 2 hours. After cooling, the reaction is poured into ice water under vigorous stirring, filtered, and the obtained solid is washed with water until the acetic acid smell has disappeared.

The solid is then heated at reflux in 70% ethanol for 4 hours under stirring.

After cooling, the reaction solution is filtered; the mother liquor is slightly concentrated, cooled in an ice bath and filtered once again. Yield: 86%; melting point: 290°C

### b) Acetyl betulinic acid chloride (MW 517.18) III

Two grams of acetyl betulinic acid II are provided in dry benzene and treated with a 10-fold excess of oxalyl chloride (3.4 ml). The reaction mixture is stirred for 8 hours under cooling, and the solvent as well as excess oxalyl chloride is subsequently evaporated on a rotary evaporator. In order to remove any oxalyl chloride residues, another 20 ml of benzene are added and again evaporated under vacuum.

### c) Acetyl betulinic acid-2-amino-3-hydroxy-2-hydroxymethyl propyl ester (MW 601.86) IV (NVX-207) (Compound B)

The acid chloride obtained from 1 g acetyl betulinic acid by the method according to b) is reacted without further purification. To this end, it is dissolved in 35 ml dioxan (dry), and tris(hydroxymethyl)-aminomethane is added in two-fold excess (0.004 mol, 0.5 g). After the addition of a spatula tip of DMAP and 3 drops of pyridine, the reaction mixture is stirred for 2 days at room temperature.

After this, the solids are filtered off; the solution is concentrated on the rotary evaporator and taken up in chloroform. This chloroform solution is washed free of pyridine with 1% hydrochloric acid, water and saturated saline solution several times. After drying over Na₂SO₄, the solvent is removed and the product is purified over a silica gel column or (and) chromatotron. A chloroform-methanol mixture at a ratio of 10:1 1 is used as an eluant. Yield: 20%, melting point: 156°C.

### 2) Acetyl betulic acid-N-(1,1-bis(hydroxymethyl)-2-hydroxyethyl)formamide (MW 601.86), (Compound C)

The synthesis of acetyl betulinic acid N-(1,1-bis(hydroxymethyl)-2-hydroxyethyl)formamide (compound C) is carried out in a manner analogous to reaction scheme 1, departing from betulic acid I via the intermediate stages of acetyl betulic acid II and the respective acid chloride III, by reacting the acid chloride III with tris(hydroxymethyl)aminomethane.

The acid chloride obtained from 1 g acetyl betulic acid (about 0.002 mol) according to the method of 1)b) is reacted without further purification. To this end, it is dissolved in 35 ml anhydrous dioxan, and an equimolar amount of tris-(hydroxymethyl)aminomethane (0.002 mol, 0.25 g) is added. After the addition of a spatula tip of DMAP and 3 drops of pyridine, the reaction mixture is heated to 80°C for 8 hrs. After this, the reaction solution is concentrated on the rotary evaporator and the residue is taken up in chloroform. This chloroform solution is washed free of pyridine with 1% hydrochloric acid, water and saturated saline solution several times. After drying over Na₂SO₄, the solvent is removed and the product is purified over a silica gel column or (and) chromatotron. A chloroform-methanol mixture at a ratio of 10:1 was used as an eluant. Yield: 15%, mp: 184°C.

### Example 2

### NVX-207 is well tolerated by human skin

A 1% topical solution of NVX-207 (200 µl total volume in 20% ethanol, 20% Tween® 80 in water) was topically applied to an area of skin on a human arm once a day for one week. A control solution (20% ethanol/20% Tween® 80 and 80 % water) was applied to an area of skin adjacent to the area to which the NVX-207 solution was applied, also for one week. The NVX-207-treated and control-treated areas exhibited no undesirable (toxic) effects during the treatment period, at the end of the treatment, as well as when checked again 6 months thereafter. Thus, NVX-207, in contrast to 5-FU or Aldara®, is well-tolerated by human skin, and does not cause any adverse acute or late reactions.

### Example 3

### Treatment of human hyperkeratotic lesions with inflammatory components

A 1% topical solution of NVX-207 (20% ethanol, 20% Tween® 80 and 80% water) was applied to human skin to treat a treatment resistant hyperkeratotic palpable lesion with inflammatory components. The NVX-207 was administered once a day on days 1, 2, 3, 10, 11, 12 and 13. Significant improvement (better than 80% reduction) was observed after one day, and additional improvement was observed after a total of six additional treatments applied within 14 days. After 14 days, the initial lesion was no longer visible or palpable. No reddening, inflammation or any other side effects were observed. A second identical lesion was treated with the same formulation twice a day for 2 days, and disappeared. No deleterious (toxic) effects on the treated areas (e.g., inflammation, redness, swelling) were observed.

### Example 4

### Treatment of canine squamous cell carcinoma with NVX-207

Since actinic keratosis can progress to squamous cell carcinoma, a study was performed using NVX-207 on a canine squamous cell carcinoma of the nasal plane. The carcinoma to which NVX-207 was applied did not respond to other modes of treatment, including surgical resection, radiation therapy and systemic chemotherapy. Histology was confirmed by biopsy before treatment. Prior to each treatment, the dog was physically examined and routine blood counts and serum chemistry analyses were performed. Tumor measurements were obtained using callipers at each treatment. Tumor size was determined by the longest diameter of the target lesion or by calculating the mean of the longest diameters of the lesion treated. Tumor response was classified as stable (less than 50 % change), partial - (decrease by > 50%) or as a complete response if no palpable or visible evidence of tumor was available. Intralesional treatment of the patient comprised local infiltration of the local tumor, including 1 cm of the surrounding tissue, with NVX-207 in combination with cisplatin. The dog was treated by a combination of cisplatin (0.33 mg/ml final concentration)/NVX-207 (3.3 mg/ml final concentration) once every three weeks for 18 weeks, resulting in complete remission. Tumor recurrence was observed 18 months later. Thus, an unexpectedly long-lasting response was achieved with the combination of NVX-207/cisplatin in which cisplatin was used at one third the concentration than would have been used if cisplatin was administered alone.

No systemic side effects were observed during the treatments. Local side effects consisted of mild local pain after infiltration of the tumor and surrounding tissue which resolved within 12 hours. No further complications were observed.

### Example 5

### Treatment of canine basal cell carcinoma; anti-inflammatory effects

A 1% topical solution of NVX-207 in 20% ethanol, 20% Tween® 80 was applied to an exophytically growing slightly bleeding (on contact) tumor on the nose of a dog using a NVX-207 soaked cotton swab every second to third day for 7 weeks. Significant improvement with an about 90 % reduction was observed after 3 weeks and bleeding stopped; except for a small prominent scar, the lesion had almost disappeared by week 7. Twenty weeks after the end of treatment no evidence of re-growth was observed. Notably, treatment effects were observed in the absence of inflammation. This shows that topical application of NVX-207 effectively treated, i.e. improved, a canine lesion clinically diagnosed as basal cell carcinoma.

### Example 6

### Activity of NVX-207 in additional canine cancer patients

NVX-207 also effectively treated a case of canine mammary carcinoma, a case of scent gland carcinoma, and two separate cases of soft tissue sarcoma one of which is summarized below. Notably, NVX-207 administration attained these treatment effects in the absence of inflammation.

Table 1 (below) shows the most important clinical parameters of three additional canine cancer patients treated intra-tumorally with NVX-207. Tumor size and treatment responses were determined as in Example 4. Intra-lesional treatment of the patients consisted of local infiltration of the tumor including 1 cm of surrounding tissue - if applicable - with NVX-207 alone. Infiltration was carried out under either general or local anaesthesia, as appropriate. As a mono-substance, NVX-207 was used at 10 mg/ml concentrations formulated with the non-toxic solubilizer 2-hydroxypropyl-γ-cyclodextrin in water. Abbreviations used are: F, female; M, male; PR, partial remission, SD, stable disease. Overall survival time is given in months. Dog number 1 in the table below is described above in Example 4.

**Table 1**

| Patient | Age, Gender | Tumor type | Treatment cycles | Tumor Response | Overall Survival |
|---|---|---|---|---|---|
| #1 | 11, F | Soft tissue sarcoma | 48 | PR | 15 |
| #2 | 12, F | Mammary carcinoma | 6 | SD | alive |
| #3 | 5, M | Sweat gland adenocarcinoma | 6 | SD | alive |

### Example 7

### Cell growth in select human and canine cancer cell lines treated with NVX-207 compared to betulinic acid

Melanoma cell lines used in this study were maintained in Dulbecco's modified Eagle medium (DMEM with 4500 mg/l glucose) supplemented with 10% FCS from Gibco BRL (Life Technologies, Berlin, Germany). The canine mastocytoma cell line C2 (DeVinney et al, Am. J. Respir. Cell Mol. Biol. 3:413-20, 1990) was used in this study, along with additional human cell lines: RD-ES and TC-71 (human Ewing's sarcoma), SAOS2 (human osteosarcoma), HT-1080 (human fibrosarcoma), Caco-2 (human colon adenocarcinoma), A549 (human lung adenocarcinoma), Hep-G2 and Hep-3B (human hepatocellular carcinoma), SK-N-DZ and SK-N-FI (human embryonal neuroblastoma), HTB9 (human bladder cancer), MDA-468 and MCF-7 (human breast cancer) and SW872 liposarcoma. All breast cancer cells were routinely cultured in DMEM/F-12 or in DMEM alone supplemented with 10% FCS at 37 °C in a humidified 5% CO₂ atmosphere. RD-ES and TC-71 sarcoma cell lines were maintained in RPMI 1640 (Biochrom KG, Berlin, Germany) containing 10% FCS at 37°C in a humidified 5% CO₂ atmosphere. Neuronal cells were maintained as monolayers in RPMI-1640 medium supplemented with 10% FCS and 1% L-glutamine under standard cell culture conditions (37 °C, 5% CO₂) in a humidified incubator. Primary human umbilical endothelial cells (HUVEC) were obtained from Lonza GmbH (Wuppertal, Germany) and cultivated according to the manufacturer's instructions. For standard *in vitro* growth experiments, cells during exponential growth phase were treated at the concentrations indicated in Figure 1.

Briefly, cells were maintained in standard medium, harvested, and plated at equal densities (750,000 cells in 5 cm tissue culture dishes). Cells were exposed to the respective treatments in fresh medium 18 hrs after plating. Determination of survival was performed after exposure to treatment by counting cell numbers in four independent dishes. Cell survival experiments were repeated at least three times for each cell line. For the assessment of cell growth, the number of surviving cells was determined using an automated cell counter from Beckman Coulter GmbH (Krefeld, Germany).

As depicted in Figures 1A-D, NVX-207 potently inhibited cell growth of melanoma, liposarcoma, colon- and lung cancer cells. Depending on the cell line, and the concentration employed, NVX-207 was several fold more effective compared to betulinic acid. Moreover, it is also of interest to note that NVX-207 survival curves exhibited a different steepness than that of betulinic acid. At concentrations of NVX-207 above 2.0 - 2.5 µg/ml (> 4.0 µM), growth of all tumor cell lines investigated was almost completely inhibited. In contrast, the parent compound betulinic acid did not fully inhibit cell growth in all four cell lines at concentrations up to 10 µg/ml. Similar results were obtained with several other cell lines including Ewing's sarcoma, osteosarcoma, fibrosarcoma, hepatocellular carcinoma, breast carcinoma, neuroblastoma, bladder cancer and mastocytoma. Thus, NVX-207 unexpectedly inhibited the growth of numerous histological types of cancer cell lines much more effectively than betulinic acid.

In order to further elucidate the pharmacodynamics of cell death induced by NVX-207, the responses of the human melanoma (518A2) cell line to NVX-207 and betulinic acid, respectively, were compared. Betulinic acid treatment did not lead to the same extent of cell death seen with NVX-207, even at an almost 5-fold higher molar concentration (Figures 2A-B).

Table 2 is a list of human and canine cancer cell lines in which NVX-207 was tested and found to be effective. Of note, all cancer cell lines tested to date responded to NVX-207 treatment. The tumor cell's TP53 mutational status is also shown. These data demonstrate that NVX-207 is also active in cell lines in which mutations in the TP53 gene occur. The concentrations at which half-maximal inhibition of cell death occurs are also given. Data marked with an asterisk (*) were obtained from the Cancer Cell Line Project IARC database (http://www.sanger.ac.uk/genetics/CGP/CellLines). For some cell lines, TP53 mutational status was deduced form the literature. For the canine cell lines, no information on mutational status is available.

**Table 2**

| Cancer cell lines | | | TP53 status | IC50 (µM) |
|---|---|---|---|---|
| Human | | | | |
| | **Melanoma** | | | |
| | | **518A2** | **mut.** | **2.6** |
| | **Lung adenocarcinoma** | | | |
| | | **A549** | **neg.*** | **2.5** |
| | **Colon adenocarcinoma** | | | |
| | | **Caco-2** | **neg.*** | **2.7** |
| | **Breast cancer** | | | |
| | | **MDA-MB-468** | **mut.** | **3.6** |
| | | **MCF-7** | **neg.*** | **4.0** |
| | **Neuronal cancer** | | | |
| | | **SK-N-DZ** | **mut.*** | **2.7** |
| | | **SK-N-FI** | **mut.*** | **3.7** |
| | | **GOTO (autonomic ganglia)** | **neg.*** | **2.0** |
| | **Hepatocellular carcinoma** | | | |
| | | **Hep-3B** | **TP53 null** | **3.8** |
| | | **Hep-G2** | **neg.*** | **3.3** |
| | **Sarcoma** | | | |
| | | **RD-ES (Ewing's sarcoma)** | **mut.*** | **5.3** |
| | | **TC-71 (Ewing's sarcoma)** | **mut.*** | **5.5** |
| | | **HAT 1080 (fibrosarcoma)** | **mut.*** | **5.6** |
| | | **SW872 (liposarcoma)** | **mut.*** | **1.8** |
| Canine | | | | |
| | **Mastocytoma - C2** | | | **3.0** |
| | **Osteosarcoma - D17** | | | **3.8** |
| | **Melanoma - TLM1** | | | **3.8** |

### Example 8

### Caspase and poly ADP ribose polymerase (PARP) cleavage in NVX-207-treated human melanoma cells

Preparation of cell extracts and protocols for the use of antibodies for Western blotting followed manufacturers' recommendations. The amount of protein used was quantified by means of a modified Bradford analysis (Bio-Rad, Richmond, CA, USA). Total lysates (10-20 µg per lane) were then separated by SDS-polyacrylamide gel electrophoresis and blotted onto nitrocellulose membranes. Bound antigen was visualized with the enhanced chemiluminescence detection system (ECL) from Boehringer Ingelheim Austria GmbH (Vienna, Austria). A cleaved caspase antibody sampler kit was obtained from Cell Signaling Technology, Inc. (Danvers, MA, USA), and includes a panel of antibodies specific for cleaved PARP (poly (ADP-ribose) polymerase). PARP cleavage was analyzed in the presence of both betulinic acid (21 µM) and NVX-207 (8.3 µM). NVX-207-induced cell death was mediated by activation of the intrinsic apoptotic pathway via cleavage of caspases 9, -3 and -7 and of PARP. PARP cleavage was shown to occur more rapidly and after treatment with lower concentrations of NVX-207 compared to betulinic acid treated cells.

### Example 9

### Effects of NVX-207 on primary target cells (endothelial cells, keratinocytes and fibroblasts) of psoriasis

Human umbilical vein endothelial cells (HUVEC), keratinocytes and fibroblast cells were obtained from Lonza GmbH (Wuppertal, Germany) and cultivated according to the manufacturer's instructions. NVX-207 exhibited greatly enhanced antiproliferative activity, compared to betulinic acid, against keratinocytes, the major cell type involved in psoriasis. The IC50 value of betulinic acid in human keratinocytes was recently determined to be ∼ 5.0 µg/ml (Tino Galgon al., Exp Dermatol. 14:736-43, 2005), while the IC50 of NVX-207 is ∼ 0.7 µg/ml. Consequently, the IC50 value of NVX-207 was almost 10-fold lower compared to betulinic acid on a molar basis. In addition, NVX-207 was also highly effective against two additional cell types important for psoriatic pathology, namely towards proliferating HUVEC as well as fibroblasts. In sub-confluent (30% confluency) rapidly proliferating endothelial cells, a 2.5 µM concentration of NVX-207 was almost 5-fold more effective compared to nearly confluent endothelial cells (80 % confluency) (Fig. 3). In a similar manner, NVX-207 was over 20-fold more active against proliferating human fibroblasts and almost 6-fold more active against keratinocytes (Figure 3). Mean survival in HUVEC at 80% confluency (black bar) was 90.3% (S.E. = 0.9) versus 18.3% (S.E. 3.9) at 30% confluency (grey bar). Mean survival in NVX-207 treated fibroblasts at 80% confluency was 92% (S.E. = 3.8) versus 3.8% mean survival (S.E. = 0.4) at 30% confluency. In keratinocytes, mean survival (at 80% confluency) was 92.7% (S.E. = 2.9) versus 17% (S.E> = 4.3) in 30% confluent cell cultures. Under normal physiological conditions, the endothelium, as well as skin fibroblasts and skin keratinocytes, show relatively low turn-over and limited cell divisions to maintain tissue integrity.

Keratinocytes at high confluence (e.g., 80%) mimic normal physiological conditions in which these cells divide more slowly (fewer cell divisions per unit of time). In contrast, keratinocytes in psoriatic lesions divide more rapidly, as do keratinocytes cultured at lower confluency (e.g., 30%). Thus, the 30% confluency conditions were considered a model for psoriasis whereas the behavior of 80% confluent cells more closely reflects the conditions observed in normal skin. NVX-207 was much more effective in keratinocytes (as well as endothelial cells and fibroblasts) at 30% confluence when these cells are actively dividing suggesting that this compound will effectively eliminate psoriatic keratinocytes, but will have little or no effect on normal keratinocytes.

### Example 10

### Topical NVX-207 treatment is well tolerated in pigs

A safety study in pigs was conducted to evaluate the potential toxicity of increasing concentrations of topically administered NVX-207. Five pigs with a mean final body mass of 16.4 kg were used for evaluating the tolerability of topically applied NVX-207. Increasing concentrations of NVX-207 (0.5 %, 1.0 % and 2.0 % in 20 % ethanol/20 % polysorbate in 80 % water) as well as vehicle controls were applied to defined areas on the animals' backs once daily over two weeks. Skin areas were visually inspected every day. Photographs were taken at three day intervals. At days 0, 7, and 14 blood samples were taken by puncture of the anterior vena cava. On day 14, pigs were anesthesized with ketamine and azaperon, and euthanized by intracardial application of T61®. No macroscopic NVX-207 related skin irritations were observed during the course of the study. Histopathological examination of skin biopsies taken on days 0 and 14 confirmed these findings. Results of blood chemistry and hematology did not reflect any systemic NVX-207-induced impact on blood status, mineral homoeostasis, and liver as well as kidney functions. No treatment- related histopathological changes were observed in biopsies of NVX-207 treated skin, and no macroscopic NVX-207 related skin irritations were observed during the course of the study. In conclusion, topical administration of NVX-207 for two weeks was well tolerated in all animals tested.

It will be understood by those of skill in the art that numerous and various modifications can be made without departing from the spirit of the present invention. Therefore, it should be clearly understood that the forms of the present invention are illustrative only and are not intended to limit the scope of the present invention.

All documents and other information sources cited above are hereby incorporated in their entirety by reference.
The present invention provides for various aspects and embodiments of the invention, including:
In a first aspect; a method of treating or preventing a disorder having an inflammatory component in a mammal in need thereof, comprising identifying a mammal in need of such treatment, and administering an effective amount of a compound having the following formula, a related compound, or a pharmaceutically acceptable salt thereof, to said mammal: 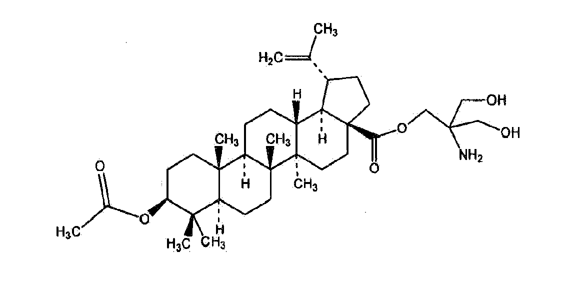
In a second aspect, the method of the first aspect, wherein said compound is administered topically.
In a third aspect, the method of the second aspect wherein said compound is formulated as a lotion, ointment, gel, cream or paste.
In a fourth aspect, the method of the second aspect wherein the compound is formulated in a transdermal delivery system.
In a fifth aspect, the method of the first aspect, wherein said compound is administered to the oral cavity.
In a sixth aspect, the method of the first aspect, wherein said compound is administered intravenously, intramuscularly, subcutaneously or via aerosol.
In a seventh aspect, the method of the first aspect, wherein said disorder is selected from the group consisting of dermatitis, rosacea, eczema, alopecia, mucositis, psoriasis and lupus.
In an eighth aspect, the method of the first aspect, wherein said mammal is a human.
In a ninth aspect, the method of the first aspect, wherein said related compound has the following general formula: 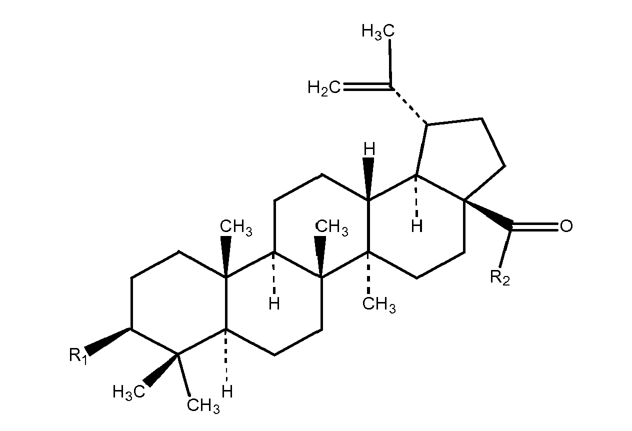 wherein R₁ represents a hydroxy group, an amino group, a protected hydroxy group or a protected amino group, and R₂ is one of the following substituents: 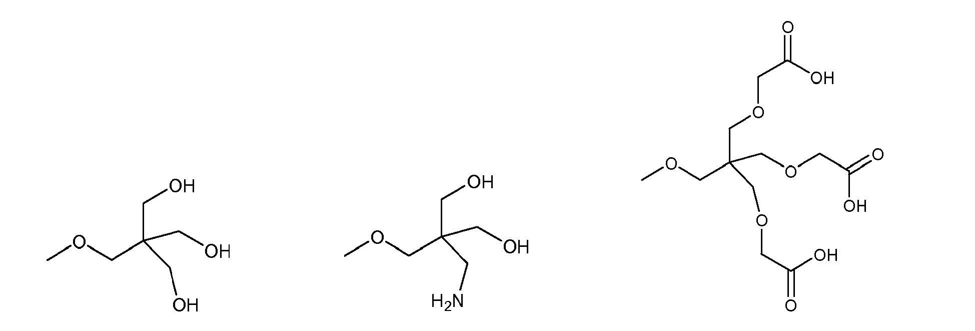 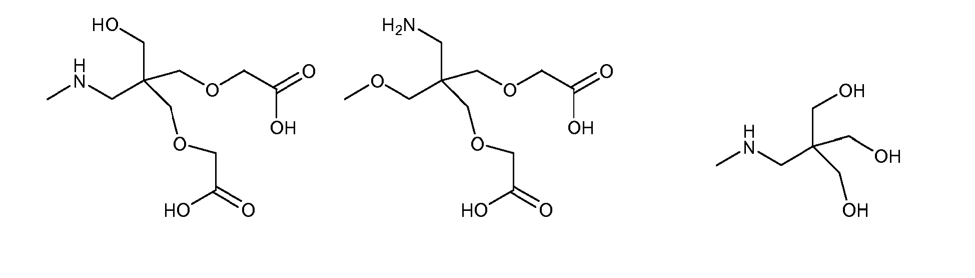
In a tenth aspect, the method of the ninth aspect, wherein R₁ represents a hydroxy group, an amino group or one of the following protected hydroxy or amino groups: 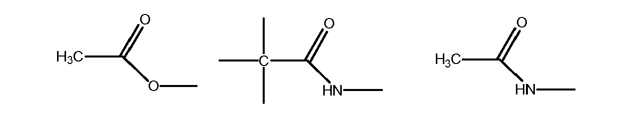 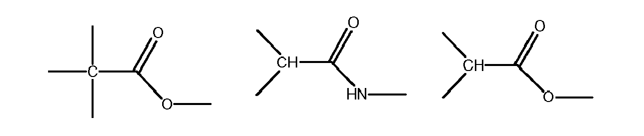 and R₂ is as defined above.
In an eleventh aspect, a pharmaceutical formulation comprising the following compound, or pharmaceutically acceptable salt thereof; ethanol and/or DMSO in an amount from 0.1- 70% (v/v); and a nonionic detergent in an amount from 0.1-70% (v/v): 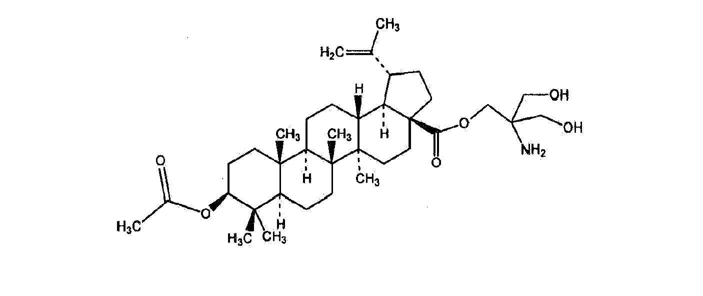
In a twelfth aspect, the pharmaceutical formulation of the eleventh aspect, wherein said nonionic detergent is selected from the group consisting of Cremophor EL® (polyethoxylated castor oil), Tween® 80 (polyoxyethylen-sorbitan-monooleate), Tween® 40 (polyoxyethylene (20)-sorbitan-monopalmitate), Triton® X-100 (polyethylenglycol-[4-(1, 1,3,3- tetramethylbutyl)phenyl] -ether), Span® 20 (sorbitan monolaurate) and Span® 85 (sorbitan trioleate.
In a thirteenth aspect, the pharmaceutical composition of the eleventh aspect, wherein said ethanol and/or DMSO is present in an amount of 20% (v/v).
In a fourteenth aspect, the pharmaceutical composition of the eleventh aspect, wherein said nonionic detergent is present in an amount of 20% (v/v).
In a fifteenth aspect, a method of treating or preventing a disorder associated with aberrant proliferation of skin or mucosal membrane keratinocytes or basal cells in a mammal in need thereof, comprising identifying a mammal in need of such treatment, and administering an amount of a compound having the following formula, a related compound, or a pharmaceutically acceptable salt thereof, to said mammal: 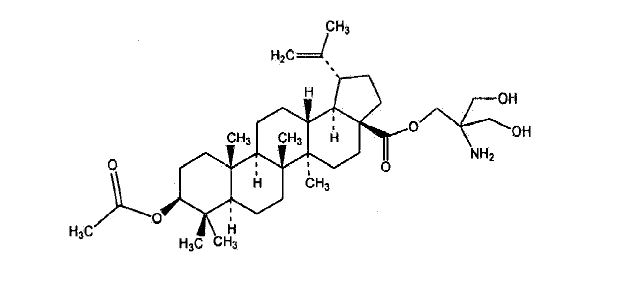
In a sixteenth aspect, the method of the fifteenth aspect, wherein said related compound has the following general formula: 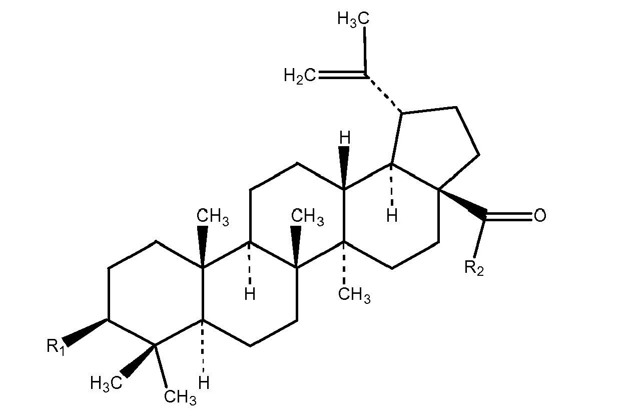 wherein R1 represents a hydroxy group, an amino group, a protected hydroxy group or a protected amino group, and R₂ is one of the following substituents: 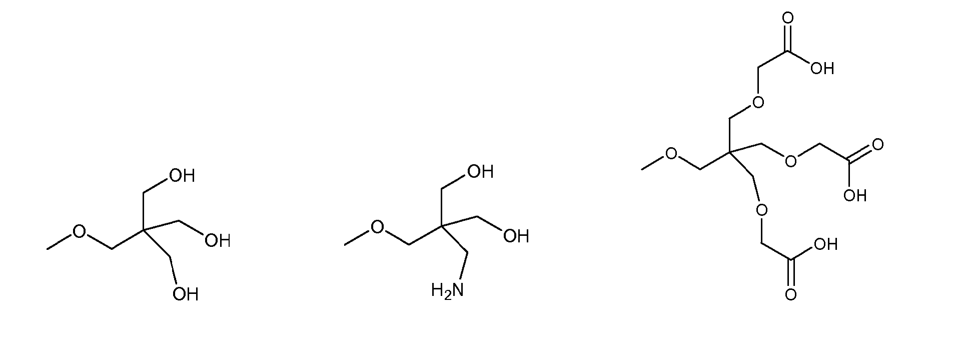 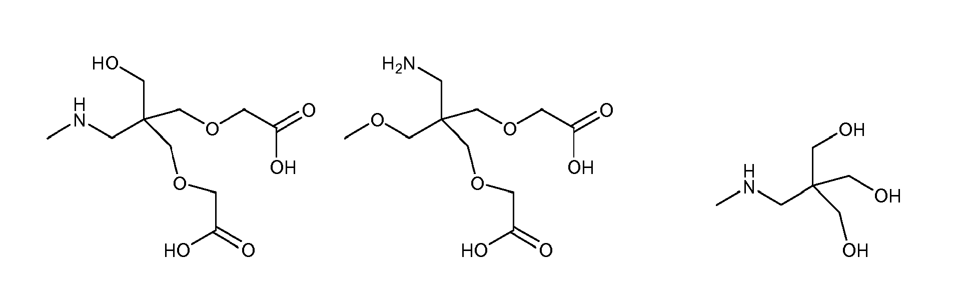
In a seventeenth aspect, the method of the sixteenth aspect, wherein Rj represents a hydroxy group, an amino group or one of the following protected hydroxy or amino groups: 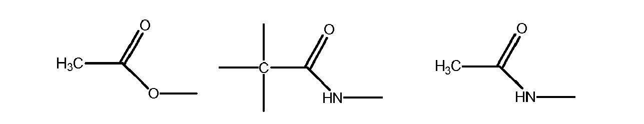 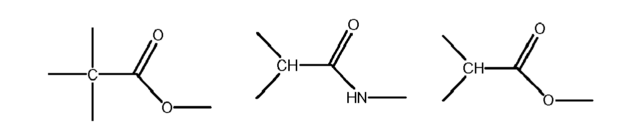 and R₂ is as defined above.
In an eighteenth aspect the method of the fifteenth aspect, wherein said disorder is selected from the group consisting of actinic keratosis, arsenical keratoses, thermal keratoses, hydrocarbon keratoses, chronic radiation keratoses, reactional keratoses, PUVA keratoses, viral keratoses, Bowenoid papulosis, epidermodysplasia verruciformis, erythroplasia of Queyrat, leukoplakia, erythroplakia, Bowen disease, squamous cell carcinoma, basal cell carcinoma and psoriasis.
In a nineteenth aspect, the method of the fifteenth aspect, wherein said disorder is a hyperkeratotic lesion.
In a twentieth aspect, the method of the fifteenth aspect, wherein said mammal is a human.
In a twenty first aspect, the method of the fifteenth aspect, wherein said compound is administered topically.
In a twenty second aspect, the method of the twenty first aspect, wherein said compound is formulated as a lotion, ointment, gel, cream or paste.
In a twenty third aspect, the method of the twenty first aspect, wherein said is formulated in a transdermal delivery system.
In a twenty fourth aspect, the method of the fifteenth aspect, wherein said compound is administered to the oral cavity.
In a twenty fifth aspect, the method of the fifteenth aspect, wherein said compound is administered intravenously, intramuscularly, subcutaneously or via aerosol
In a twenty sixth aspect, the method of the eighteenth aspect, wherein said disorder is actinic keratosis.
In a twenty seventh aspect, the method of the eighteenth aspect, wherein said disorder is basal cell carcinoma.
In a twenty eighth aspect, the method of the eighteenth aspect, wherein said disorder is squamous cell carcinoma.

## Claims

1. A compound having the following formula, or a pharmaceutically acceptable salt thereof, for treating or preventing a disorder associated with aberrant proliferation of keratinocytes in the skin or mucous membranes, or associated with aberrant proliferation of basal cells in the skin or mucous membranes, excluding squamous cell carcinoma, in a mammal in need thereof: 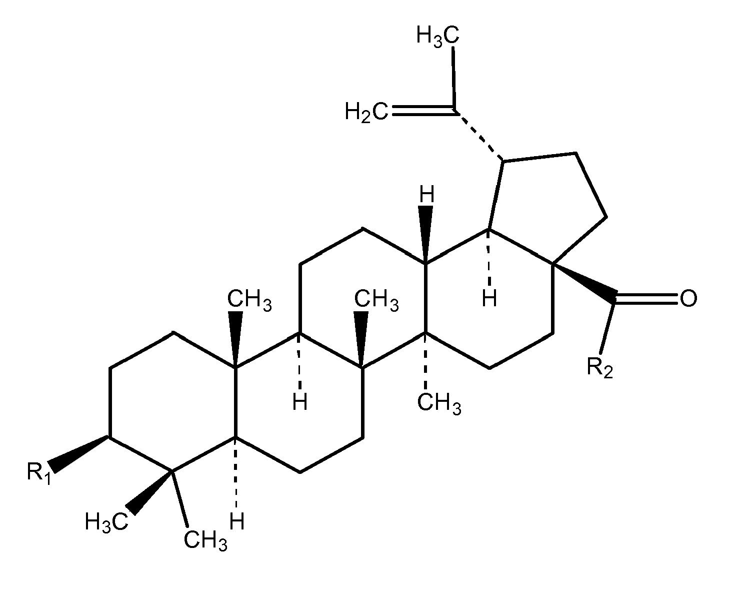 wherein R₁ represents a hydroxy group, an amino group, a protected hydroxy group or a protected amino group,
and R₂ is one of the following substituents:

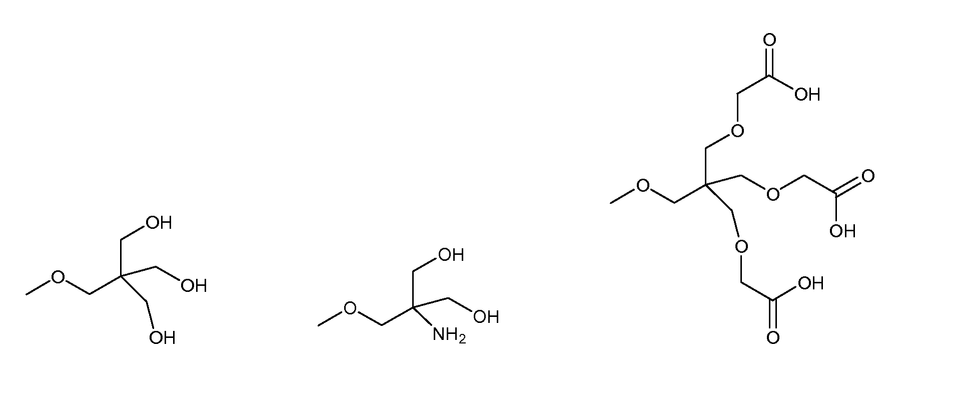

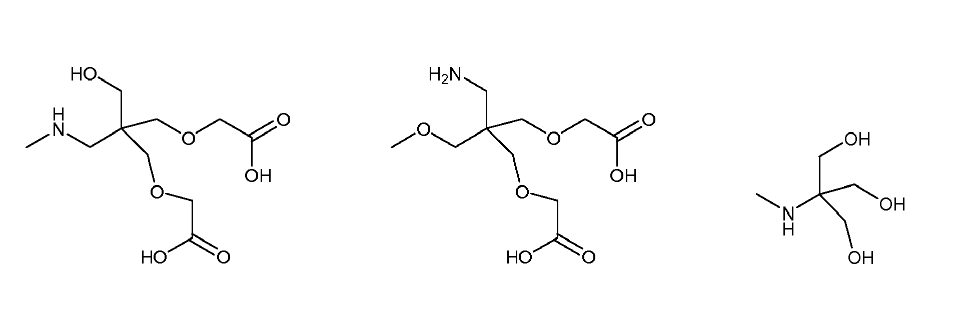

2. The compound of claim 1, wherein the disorder is keratosis, psoriasis or basal cell carcinoma.

3. The compound of claim 2, wherein the disorder is actinic keratosis, psoriasis or basal cell carcinoma.

4. The compound of claim 2, wherein the disorder is keratosis or psoriasis.

5. The compound of claim 4, wherein the disorder is actinic keratosis or psoriasis.

6. The compound of claim 5, wherein the disorder is actinic keratosis.

7. The compound of claim 5 wherein the disorder is psoriasis.

8. The compound of claim 3, wherein the disorder is basal cell carcinoma.

9. The compound of any of the preceding claims, wherein wherein R₁ represents a hydroxy group, an amino group or one of the following protected hydroxy or amino groups: 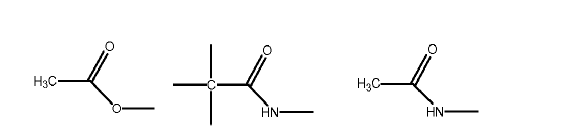 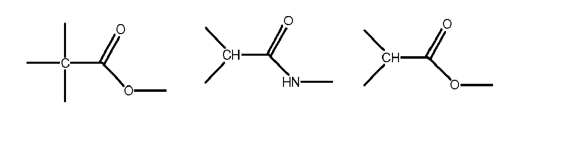 and R₂ is as defined above.

10. The compound of any of the preceding claims, wherein the compound is:

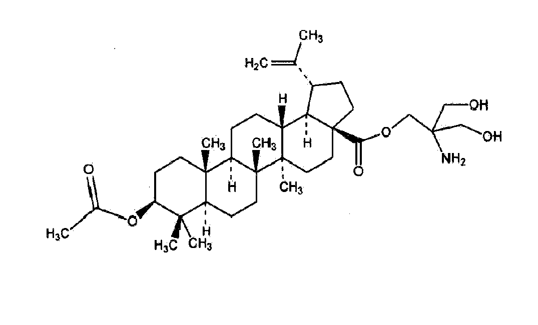

11. The compound of any of the preceding claims, wherein the compound is administered topically.

12. The compound of claim 11, wherein the compound is formulated as a lotion, ointment, gel, cream, paste or transdermal delivery system.

13. The compound of any of the preceding claims, wherein said mammal is a human.

14. A pharmaceutical formulation comprising the compound of any of the preceding claims and a pharmaceutical diluent or carrier.
